# EUROPEAN PATENT SPECIFICATION

(11) **EP 1 324 726 B1**
(45) Date of publication and mention of the grant of the patent: **09.08.2006**
(21) Application number: 01967912.5
(22) Date of filing: 19.09.2001
(51) Int. Cl.: A61F 2/64

(54) **KNEE JOINT PROSTHESIS**
KNIEGELENKPROTHESE
PROTHESE POUR ARTICULATION DU GENOU

(30) Priority: 20.09.2000 US 667052
(43) Date of publication of application: 09.07.2003
(73) Proprietor: GRAMTEC INNOVATION AB, S-511 56 Kinna (SE)
(72) Inventor: GRAMNÄS, Finn, S-511 56 Kinna (SE)
(74) Representative: Egeröd, Lisbeth
(86) International application number: PCT/SE2001/002013
(87) International publication number: WO 2002/024123

(56) References cited:
- WO-A1-99/55261
- US-A- 6 033 440

## Description

### Technical field

The present invention refers to a knee joint prosthesis comprising two rotatably interconnected members the first of which carrying a braking device which via a pivot axle is rotatably connected to one of said interconnected members, said braking device when inactivated is arranged to permit said interconnected members to rotate with respect to each other but upon activation is arranged to prevent rotation from extended to bent position of the knee prosthesis, said knee joint prosthesis further comprises brake activating means for activation of the braking device, said braking device presenting a geometrical centre, said pivot axle being located off-centre with respect to said geometrical centre of the braking device, said knee joint prosthesis further comprising an upper socket adapted for connection to an upper leg prosthesis or prosthesis sleeve and a lower socket adapted for connection to a lower leg prosthesis.

### Background of the invention

Traditional friction locks in knee prostheses will lock when there is a load acting on them, i e when some part of the body weight rests on the artificial knee. This takes place in a still standing position as well as during the walking phase at heel strike when the heel hits the ground, and during toe off supporting oneself on the toe while extending the leg to initiate the swing phase when the leg swings freely in the air.

At normal walking without a prosthesis one starts already during toe off to flex the knee-joint to initiate the swing phase before all body weight is has been removed from the leg in question. This is not possible with knee prostheses with a friction lock of the conventional kind. This involves an unnatural walking and makes walking in stairs and broken ground and cycling difficult since the knee prosthesis will lock as soon as it is loaded.

Knee prostheses with friction locks in the form of a brake drum are disclosed in US-A-4,206,519 and 4,351,070. In the last mentioned document there is a linkage transferring motions in the hip axis to the locking device in order to control the locking function thereof in response to the torque of the hip axis.

WO 97/10781 discloses a knee prosthesis with a friction lock in the form of a resiliently deformable substantially C-shaped member cooperating with an axle. The C-shaped member can be rotated about the axle in unloaded position, but in a locked position be deformed and locked to the axle.

US Patent Number US 6,033,440 describes an adjustable pyramidal link plate assembly for a prosthetic limb. The assembly allows infinite rotatable adjustment without significantly increasing the profile or length of the limb and without requiring the limb to be removed or disassembled during adjustment.

WO 99/55261 discloses a knee joint prosthesis of the kind stated in the introductory part above. The important advantage of this knee joint is that it permits rotation of the knee-joint in unloaded position and when it still is under body load during toe off when supporting oneself on the toe while extending the leg to initiate the swing phase, but which locks against rotation from extended to flexed position at other loaded positions, i e during heel strike when supporting oneself on the heel and during mid stance when supporting oneself on the whole foot.

### The subject and most important features of the invention

It is an object of the invention to provide a knee prosthesis of the kind disclosed in WO 99/55261 and wherein it is possible to adjust where in the walking cycle the locking device is activated and released respectively. This has according to the invention been achieved by the fact that said upper and lower socket each include angularly adjustable connection means. By this the geometrical balance of the knee joint can be adjusted which will bring about the desired function.

According to one embodiment that the angularly adjustable connection means comprises a frustopyramidal member the angularly adjustable connection means comprises a frustopyramidal member

According to a further embodiment the angularly adjustable connection means comprises a male member having a substantially spherically convex base and a boss portion, the substantially spherically convex base being adapted to attach to the knee prosthesis, said angularly adjustable connection means further comprises a female member in the form of an annular socket adapted to retain the boss portion, the annular socket further being adapted to connect to a prosthesis sleeve and/or a leg prosthesis and means for adjustable retainment of the two members in a selected angular position.

Preferably said means for adjustable retainment comprises a plurality of set screws extending through the surface of the annular socket to engage the surface of the boss portion retained therein.

It is further preferred that the boss portion has a frustopyramidal shape.

According to a further embodiment the braking device comprises a brake shoe in the shape of an open ring and thus forming a pair of shanks and cooperating with a brake activating lever arm extending between said shanks and at activation of the braking device forces said shanks apart, wear adjustment means being attached to said brake activating arm. Preferably said wear adjustment means comprises a spring-loaded wedge.

### Description of drawings

The invention will below be described more in detail with reference to some embodiments shown in the accompanying drawings.
Fig. 1a and b are longitudinal sections through an embodiment of the knee prosthesis in locked and unlocked positions.
Fig. 2a and b illustrate schematically the brake activating mechanism in non-activated and activated position respectively.
Fig. 3 a and b are schematic views illustrating the principle of function of the knee prosthesis.

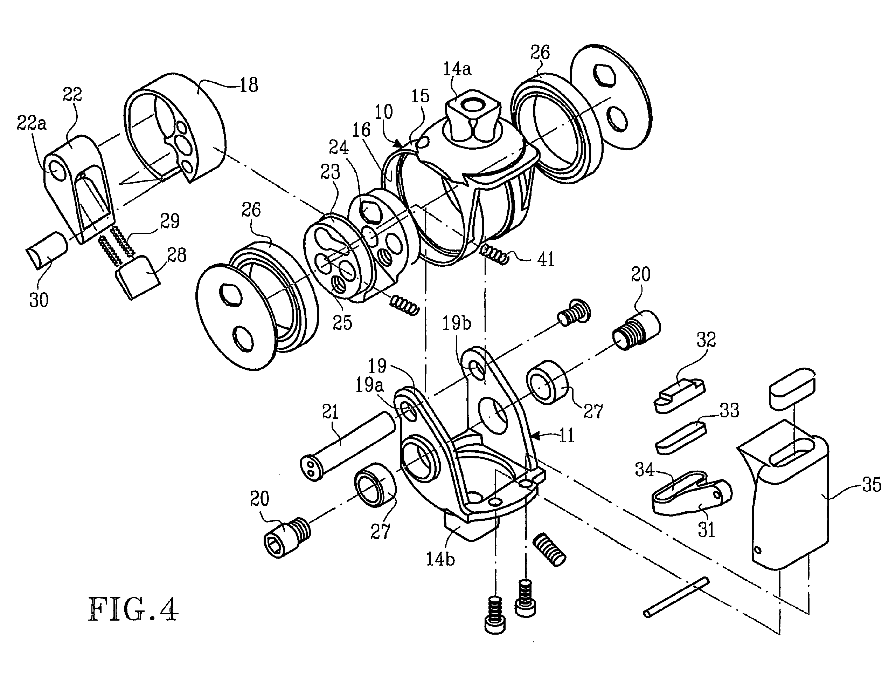

Fig. 4 is an exploded view of the knee prosthesis according to Fig. 1.

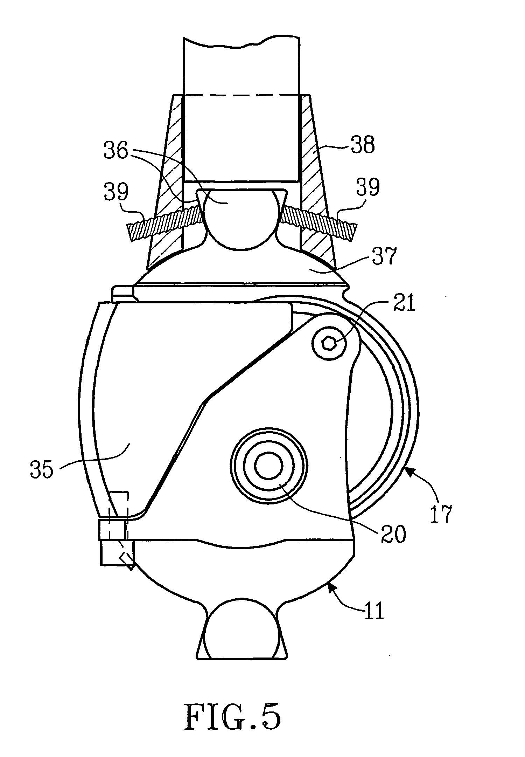

Fig. 5 is a side view of the knee prosthesis according to Figs. 1 and 2 and showing its attachment to a leg prosthesis.
Fig. 6 a and b show in a toe off position the knee prosthesis according to Fig. 4 connected to upper and lower leg prostheses in two different angularly adjusted positions.
Fig. 7 a-c illustrates the function of the knee prosthesis during the different phases of the walking cycle.

### Description of embodiments

The knee prosthesis according to the invention comprises an upper and a lower member 10 and 11 which are rotatably interconnected. The upper member is intended to be attached to a prosthesis sleeve 12 or a upper leg prosthesis and the lower member to a lower leg prosthesis 13 having a foot prosthesis 13a connected thereto.

The upper knee prosthesis member 10 comprises a socket 14a for connection to a prosthesis sleeve or upper leg prosthesis, and a housing portion 15 with a through opening 16 for receiving a braking device 17 comprising a brake shoe 18. The lower knee prosthesis member 11 is provided with a socket 14b for connection to a lower leg prosthesis 13, and a yoke-shaped upper portion 19 between the shanks 19 a and b of which the housing portion 15 of the upper knee prosthesis member 10 is received.

The brake shoe 18 is received between a pair of interconnected plates 23 received in the housing 15. The interconnected plates 23 together with the brake shoe 18 form the braking device 17 presenting a part-cylindrical outer surface.

Two axles 20 and 21 extend substantially in parallel through the shanks 19. The first axle 20, which in the embodiment shown is divided in two parts, is rotatably supported in said shanks 19 of the second member 11. The brake shoe 18 and the interconnected plates 23 are mounted to the first axle 20, which thus forms a pivot axle for the braking device. The second axle 21 is received in the open space in the brake shoe 18 and forms a part of the activation mechanism for the braking device as will be described more in detail below. The second axle 21 is arranged radially displaced from the pivot axle 20.

Bearing means 26 such as a ball bearing may be arranged on both sides of the brake shoe 18. By this the lateral stability of the knee joint is improved. The interconnected plates 23 have an oblong hole 24 for permitting the brake-activating movement with respect to the second axle 21 and is further provided with threaded holes 25 for receiving corresponding threaded connections of the first axle 20. The numeral 27 denotes bearing members such as needle bearings.

A brake activating member 22 in the form of a lever arm 22 is further provided. The brake shoe 18 has the shape of an open ring and the lever arm 22 extends in between the shanks 17a and b of the brake shoe. The brake activating lever arm 22 is provided with an opening 22a through which the second axle 21 extends.

The lever arm 22 is further provided with an automatic wear adjustment means in the form of a spring-loaded wedge 28. As the brake shoe 17 is worn on the outside the gap between its shanks is increased. The wedge 28 is then by the springs 29 pressed further into the gap between said shanks, so that there will not be needed a stronger force to activate the brake function. The numeral 30 denotes a member applied in a recess in the brake shoe and arranged to provide a contact surface for the wedge 28.

The lever arm 22 causes the brake shoe 18 to expand and to be friction locked against the inside of the opening 16 forming a brake drum. A friction adjustment device, with which the friction resistance against rotation of the knee joint is adjusted is arranged and comprises a brake arm 31, which can be tightened against the part-cylindrical surface 10a of the upper knee joint member 10. A friction element 32 rests against a resilient rubber element 33 is arranged in a recess 34 in the brake arm 31. The brake arm 31 is so arranged with respect to the axles 20 and 21 that the friction element 32 will press against the knee joint member 10 at a point just opposite the axle 20 and its connection line with the axle 21. By this the important advantage is obtained that the friction in the knee joint can be adjusted without in a corresponding way influencing the function of the braking mechanism, i e the axle 21, the lever arm 22 and the brake shoe 18. The brake arm 31 is connected to a housing portion 35.

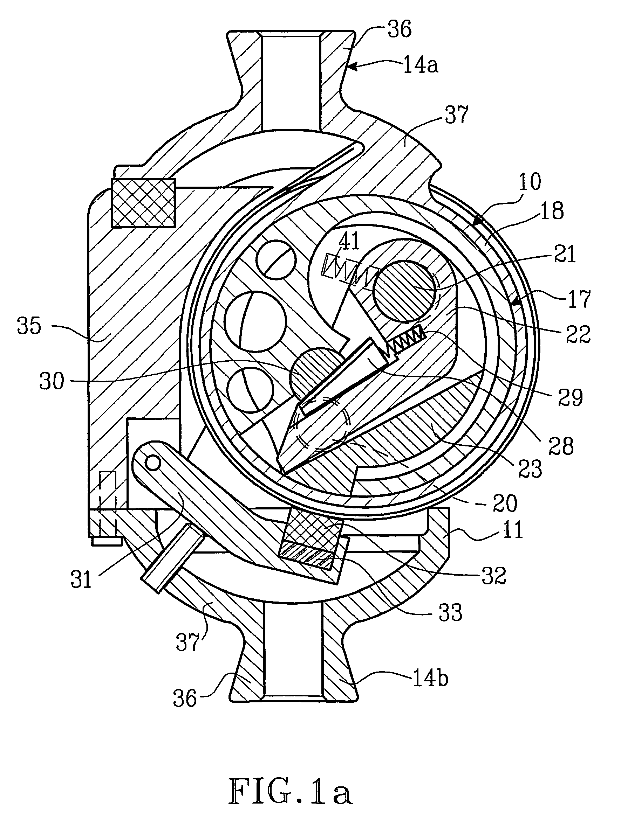

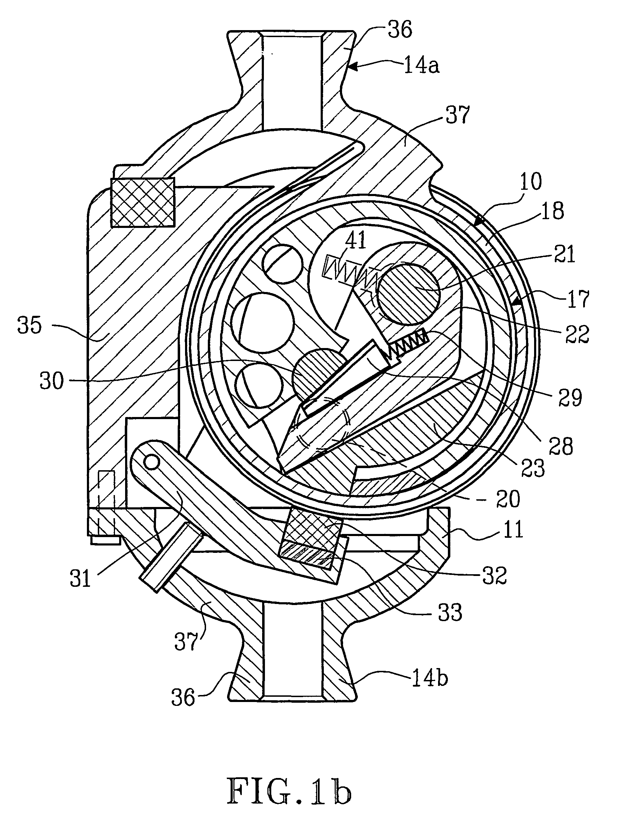

The knee joint is shown in a braked (locked) position in Fig. 1a and in a non-braked position in Fig. 1b.

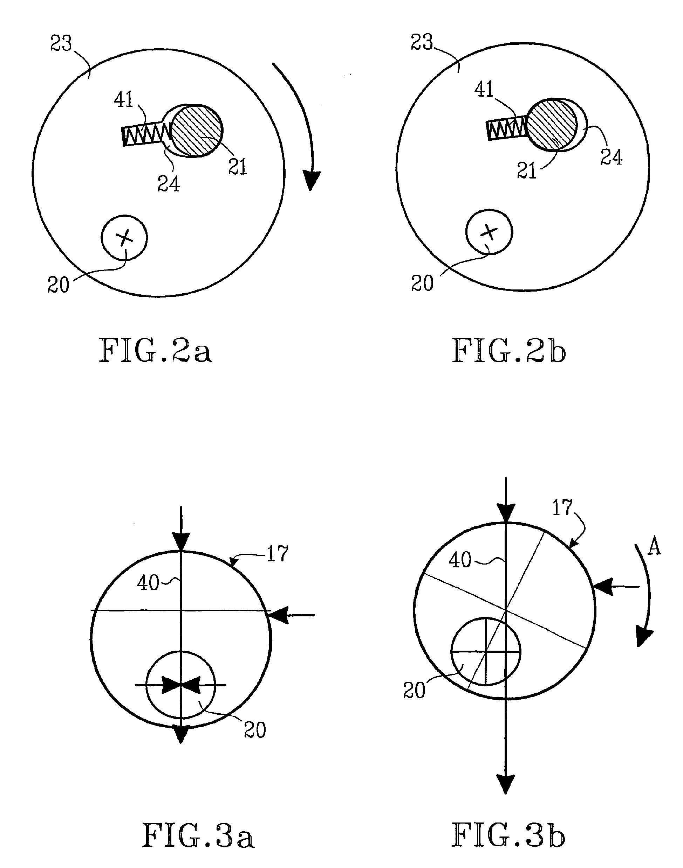

A pair of springs 41 provides a resilient element for lifting the brake activating axle 21. The springs 41 prevent the knee joint from self-locking and only lock in a brake activated position. This is illustrated i for example Fig. 2 a and b, wherein Fig. 2a shows the brake activating axle 21 in a non-activated postion and Fig. 2b in an activated position.

The principal of function of the braking system for the knee joint is schematically illustrated in Fig. 3 a and b. The principle for the geometric sensitivity of the braking device is that the internal rotatable cylindrical parts, including the brake shoe 18 and interconnected plates 23, is rotatably mounted in a rotary point, i e pivot axle 20, placed out of centre position with respect to said internal rotatable cylindrical parts 18, 23. When as illustrated in Fig. 3a the knee joint is loaded so that the line of action of the load passes through the centre of the internal cylindrical parts 18, 23 and the centre of the rotary point, i e first axle 20, the internal cylindrical part will be in balance and nothing will happen. This means that the brake is not activated.

If however as illustrated in Fig. 3b the knee joint is loaded so that the line of action of the load passes out of centre of the internal cylindrical parts 18, 23 and the centre of the rotary point, i e first axle 20, the cylindrical part will be out of balance and cannot hold the load without creating a rotary motion, which is used to activate the brake. When the brake shoe 18 is rotated it will meet the lever arm 22 which causes the brake shoe 18 to expand and to be friction locked against the brake drum formed by the opening 16 in the housing portion 15. The knee joint is by this locked.

If the knee joint is rotated from flexed to extended position, i e in the opposite direction, during loading, the friction between the inside of the opening 16 in the housing portion 15 and the brake shoe 18 will force this to rotate back to the position for unloaded position. The knee joint can then be rotated freely in counter clockwise direction. It will however immediately lock again if it during loading will be rotated in clockwise direction.

This geometric sensitivity of the braking device can, in combination with an angularly adjustable upper and lower connection to an artificial leg member or prosthesis sleeve, be utilized to adjust the knee joint so that it locks (brakes) and unlocks respectively in different phases of the walking cycle. An example of such an angularly adjustable connection is a frustopyramidal socket 36. A frustopyramidal socket of this kind is per se known from e g US 3,659,294.

The connection means comprises a male part having a spherically convex base 37 from which a substantially frustopyramidal boss 36 rises divergently within an annular socket 38 constituting the female part and to which an artificial leg member 38 is connected. The frustopyramidal boss 36 is preferably four-sided and has two pairs of contact surfaces represented by the respective sides of the frustopyramid. The annular socket 38 is provided with four adjustable abutment means in the form of setscrews 39 engaging the contact surfaces of the boss 36 for retaining the two parts in a selected angular position within predetermined swing ranges in these two parts.

By adjusting the angular position of the knee joint with respect to the upper and lower leg prostheses the geometrical balance of the knee joint is adjusted. By this the brake system is activated and released in different phases of the walking cycle. The normal position is to have the brake activated during heel strike and mid stance, but released during toe off as explained above. Some prosthesis wearer having a high demand of mobility, e g for performing sports, however prefer a less stable knee joint which locks only at heel strike and unlocks already at mid stance. Other prosthesis wearer prefers a more stable knee joint which remains locked through heel strike, midstance and toe load.

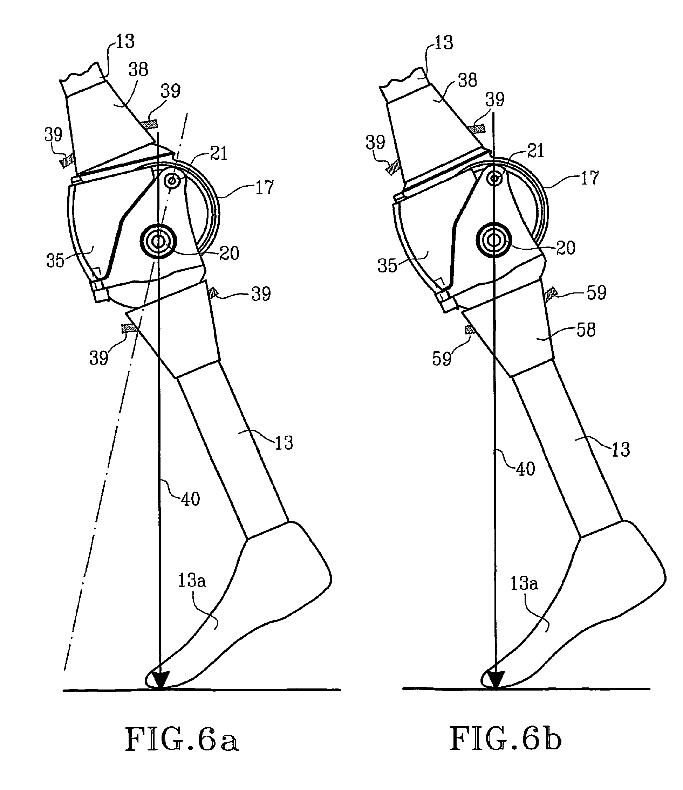

This is illustrated in Figs. 6 a and b, where Fig. 6a shows a position where the knee joint has been tilted backward as compared to the "normal" position shown in Fig. 6b. With the knee joint tilted to the position shown in Fig. 6a the brake will be activated at the toe load position shown, because the body weight line 40 will pass through the knee joint outside the centre of the rotating inner parts 18, 23 of the knee joint and their rotation axle 20. The knee joint will be out of balance and cannot hold the load without creating a rotary motion, which is used to activate the brake. With dotted lines is shown the imaginary angle of the load line needed for reaching the balanced position of the knee joint in which the brake is not activated. The knee joint remains thus locked during the entire walking phase from heel strike, midstance to toe off and is only released when there is no load on it.

In Fig. 6b is shown the "normal" position of the knee joint in which the brake is not activated in toe load position, since the body weight line 40 will pass through the geometrical centre of the rotating inner parts 18, 23 of the braking device and through the rotation axle 20. However the knee joint will be locked at heel strike and midstance.

In a corresponding manner by tilting the knee joint forwards with respect to the "normal" position shown in Fig. 6b, the knee joint can be made less stable, for example so that it releases already at midstance and locks only at heel strike.

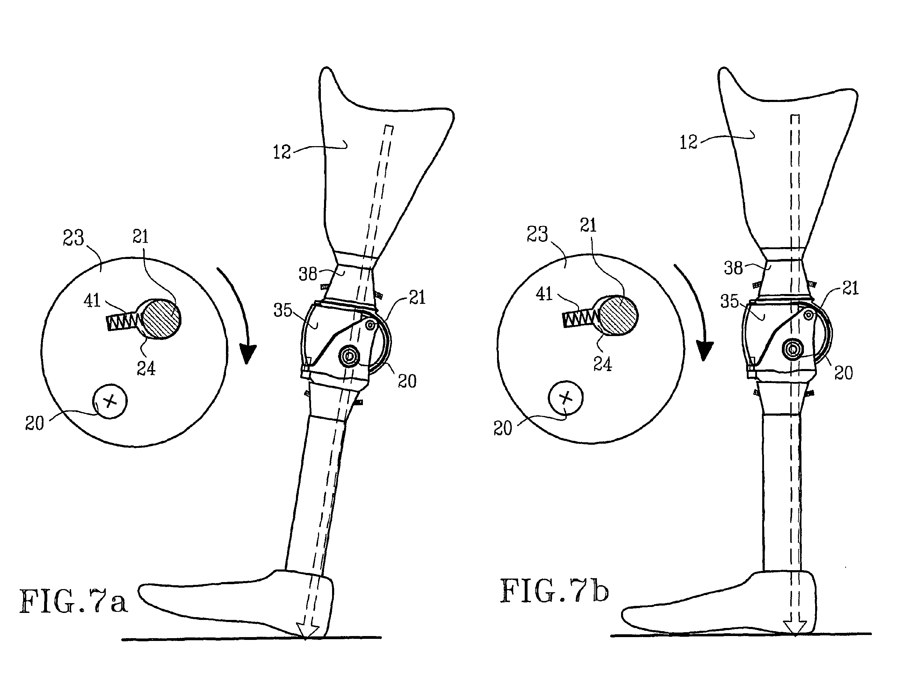

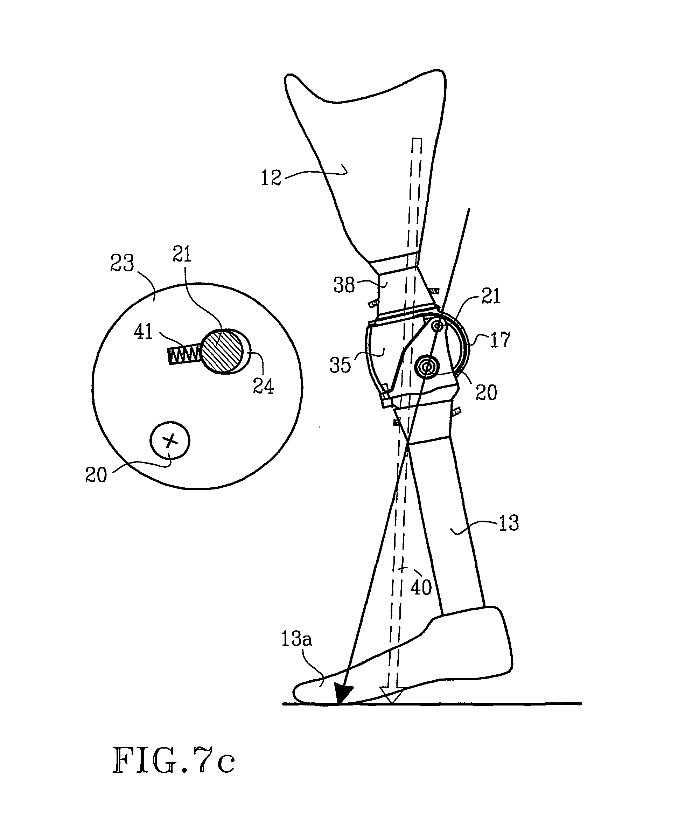

In Fig. 7 a-c the braking function of the knee prosthesis is shown during the different phases of the walking cycle. In Fig. 7a the heel strike position is shown, in which the heel strikes the ground and the wearer starts to put load on the leg in question. The body weight line illustrated by the arrow 40 in this position passes behind the geometrical centre of the rotating inner parts 18, 23 of the braking device, which means that the body weight will force the brake shoe 18 to rotate and thus activate the braking device as disclosed above.

In Fig. 7b is shown the mid stance position when the whole foot rests against the ground and the body weight acts essentially right through the leg in parallel therewith. The body weight line 40 passes also in this position behind the geometrical centre of the rotating inner parts 18, 23 and the braking device is activated.

In Fig. 7c is shown the toe off phase when supporting oneself on the toe while extending the leg to initiate the swing phase with the leg swinging freely in the air. A part of the body weight rests in this position still upon the leg. At normal walking a flexing of the knee joint takes is initiated already in this position in order to initiate the swing phase before all body weight has been removed from the leg in question. The body weight line 40 passes in this position from the toe part of the foot prosthesis 13a through the geometrical centre of the rotating inner parts 18, 23. A balanced position of the knee joint is thus reached in which the brake is not activated. The knee joint is free to rotate for initiating the swing phase of the walking cycle.

All described embodiments have a freewheel effect in such a way that the knee joint can always rotate from flexed to extended position also under load. By this for example walking in stairs will be possible. The knee-joint will however lock immediately again if it is rotated in the opposite direction, i e from extended to flexed position, under such load that the locking device is activated.

The invention is of course not limited to the embodiments shown in the drawings but can be modified within the scope of the following claims. The angularly adjustable connection means 14a and b may of course be applied in any of the embodiments of the knee joint prosthesis discloses in WO 99/55261.

## Claims

1. A knee joint prosthesis comprising two rotatably interconnected members (10,11), the first of which carrying a braking device (18, 23) which via a pivot axle (20) is rotatably connected to one of said interconnected members (10, 11), said braking device when inactivated is arranged to permit said interconnected members (10, 11) to rotate with respect to each other but upon activation is arranged to prevent rotation from extended to bent position of the knee prosthesis, said knee joint prosthesis further comprises brake activating means (21,22) for activation of the braking device (17), said braking device presenting a geometrical centre, said pivot axle (20) being located off-centre with respect to said geometrical centre of the braking device, said knee joint prosthesis further comprising an upper socket (14a) adapted for connection to an upper leg prosthesis or prosthesis sleeve (12) and a lower socket (14b) adapted for connection to a lower leg prosthesis (13),
**characterized in**
**that** the upper and lower socket (14a, b) each include angularly adjustable connection means (36-39), allowing the knee prosthesis to be tilted with respect to the upper leg prosthesis or prosthesis sleeve (12) and to the lower leg prosthesis (13).

2. A knee prosthesis according to claim 1,
**characterized in**
**that** the angularly adjustable connection means comprises a frustopyramidal member (36).

3. A knee prosthesis according to claim 1 or 2,
**characterized in**
**that** the angularly adjustable connection means comprises a male member having a substantially spherically convex base (37) and a boss portion (36), the substantially spherically convex base being adapted to attach to the knee prosthesis, said angularly adjustable connection means further comprises a female member in the form of an annular socket (38) adapted to retain the boss portion, the annular socket further being adapted to connect to a prosthesis sleeve and/or a leg prosthesis and means for adjustable retainment (39) of the two members in a selected angular position.

4. A knee prosthesis according to claim 3,
**characterized in**
**that** said means for adjustable retainment (39) comprises a plurality of set screws extending through the surface of the annular socket (38) to engage the surface of the boss portion (36) retained therein.

5. A knee prosthesis according to claim 3 or 4,
**characterized in**
**that** said boss portion (36) has a frustopyramidal shape.

6. A knee prosthesis according to any of the preceding claims,
**characterized in**
**that** the braking device (17) comprises a brake shoe (18) in the shape of an open ring and thus forming a pair of shanks (18a,b) and cooperating with a brake activating means in the form of a lever arm (22) extending between said shanks (18a,b) and at activation of the braking device forces said shanks apart, wear adjustment means (28, 29) being attached to said brake activating lever arm (22).

7. A knee prosthesis according to claim 6,
**characterized in**
**that** said wear adjustment means comprises a spring-loaded (29) wedge (28).

8. A knee prosthesis according to claim 6 or 7,
**characterized in**
at least one spring (41) arranged to act upon said brake activating means (21,22) to hold the lever arm (22) away from brake activating position.

## Patentansprüche

1. Kniegelenkprothese, welche zwei drehbar mit einander verbundene Elemente (10, 11) aufweist, von denen das erste eine Bremsvorrichtung (18, 23) trägt, welche über eine Schwenkachse (20) drehbar mit einem der mit einander verbundenen Elemente (10, 11) verbunden ist, wobei die Bremsvorrichtung im inaktiven Zustand so angeordnet ist, dass sie eine Drehung der miteinander verbundenen Elemente (10, 11) relativ zueinander ermöglicht, jedoch nach Aktivierung so angeordnet ist, dass sie eine Drehbewegung von einer gestreckten Position der Knieprothese in eine abgebogene Position derselben verhindert, wobei die Kniegelenkprothese des Weiteren eine Aktivierungseinrichtung (21, 22) für die Bremse zum Aktivieren der Bremsvorrichtung (17) aufweist, wobei die Bremsvorrichtung einen geometrischen Mittelpunkt aufweist, wobei die Schwenkachse (20) außermittig, bezogen auf den geometrischen Mittelpunkt der Bremsvorrichtung, angeordnet ist, und wobei die Kniegelenkprothese außerdem eine obere Pfanne (14a) aufweist, die zur Verbindung mit einem Oberschenkel-Prothesenteil oder einer Prothesenhülse (12) ausgelegt ist, sowie eine untere Pfanne (14b) zur Verbindung mit einem Unterschenkel-Prothesenteil (13),
**dadurch gekennzeichnet,**
**dass** die obere Pfanne und die untere Pfanne (14a, b) jeweils eine im Winkel verstellbare Verbindungseinrichtung (36 - 39) aufweisen, welche eine Kippbewegung der Knieprothese bezüglich des Oberschenkel-Prothesenteils oder der Prothesenhülse (12) und bezüglich des Unterschenkel-Prothesenteils (13) ermöglichen.

2. Knieprothese nach Anspruch 1,
**dadurch gekennzeichnet,**
**dass** die im Winkel verstellbare Verbindungseinrichtung ein pyramidenstumpfförmiges Teil (36) aufweist.

3. Knieprothese nach Anspruch 1 oder 2,
**dadurch gekennzeichnet,**
**dass** die im Winkel verstellbare Verbindungseinrichtung ein Patrizenteil mit einer im Wesentlichen sphärisch konvexen Basis (37) und mit einem Teil in Form einer Erhebung (36) aufweist, wobei die im Wesentlichen sphärisch konvexe Basis zur Anbringung an der Knieprothese angepasst ist, wobei die im Winkel verstellbare Verbindungseinrichtung des Weiteren ein Matrizenteil in Form einer ringförmigen Pfanne (38) aufweist, das so ausgelegt ist, dass es die Erhebung hält, wobei die ringförmige Pfanne außerdem zur Verbindung mit einer Protheselihülse und/oder einer Beinprothese ausgelegt ist, und eine Einrichtung zum verstellbaren Halten (39) der beiden Teile in einer ausgewählten Winkelstellung vorgesehen ist.

4. Knieprothese nach Anspruch 3,
**dadurch gekennzeichnet,**
**dass** die Einrichtung zum einstellbaren Halten (39) eine Vielzahl von Einstellschrauben aufweist, die sich durch die Oberfläche der ringförmigen Pfanne (38) so erstrecken, dass sie mit der Oberfläche der darin gehaltenen Erhebung (36) in Eingriff kommen.

5. Knieprothese nach Anspruch 3 oder 4,
**dadurch gekennzeichnet,**
**dass** die Erhebung (36) die Form eines Pyramidenstumpfs aufweist.

6. Knieprothese nach einem der vorhergehenden Ansprüche,
**dadurch gekennzeichnet,**
**dass** die Bremsvorrichtung (17) eine Bremsbacke (18) in Form eines offenen Rings aufweist und somit ein Paar Klauen (18a, b) bildet und mit einer Aktivierungseinrichtung für die Bremse in Form eines Hebelarms (22) zusammenwirkt, der sich zwischen den Klauen (18a, b) erstreckt und bei Aktivierung der Bremsvorrichtung die Klauen auseinander zwingt, wobei eine Einrichtung zur Abriebnachstellung (28, 29) an dem Hebelarm (22) zur Bremsaktivierung angebracht ist.

7. Knieprothese nach Anspruch 6,
**dadurch gekennzeichnet,**
**dass** die Einrichtung zur Abriebnachstellung einen federgespannten (29) Keil (28) aufweist.

8. Knieprothese nach Anspruch 6 oder 7,
**dadurch gekennzeichnet,**
**dass** mindestens eine Feder (41) so angeordnet ist, dass sie auf die Aktivierungseinrichtung (21, 22) für die Bremse so einwirkt, dass sie den Hebelarm. (22) aus der Position zur Bremsaktivierung hält.

## Revendications

1. Prothèse pour articulation du genou comprenant deux membres reliés l'un à l'autre par rotation (10, 11), le premier membre portant un dispositif de freinage (18,23) qui, par l'intermédiaire d'un axe de pivot (20), est relié par rotation à l'un desdits membres reliés l'un à l'autre (10,11), ledit dispositif de freinage lorsqu'il est inactivé est agencé de façon à permettre aux dits membres reliés l'un à l'autre (10,11) de tourner l'un par rapport à l'autre, mais après activation est agencé de façon à empêcher la rotation de la prothèse du genou et de passer d'une position étirée à fléchie, ladite prothèse pour articulation du genou comprenant en outre un moyen d'activation du freinage (21,22) destiné à activer le dispositif de freinage (17), ledit dispositif de freinage présentant un centre géométrique, ledit axe de pivot (20) étant excentré par rapport au dit centre géométrique du dispositif de freinage, ladite prothèse pour articulation du genou comprenant en outre un fourreau supérieur (14a) adapté pour être reliée à une prothèse de jambe supérieure ou manchon de prothèse (12) et un fourreau inférieur (14b) adaptée pour être reliée à une prothèse de jambe inférieure (13),
**caractérisée en ce que**
le fourreau supérieur et le fourreau inférieur (14a, 14b) contiennent chacun un moyen de connexion ajustable de façon angulaire (36 à 39), permettant à la prothèse du genou de basculer par rapport à la prothèse de jambe supérieure ou manchon de prothèse (12) et à la prothèse de jambe inférieure (13).

2. Prothèse pour genou selon la revendication 1,
**caractérisée en ce que**
le moyen de connexion ajustable de façon angulaire comprend un membre frusto-pyramidale (36).

3. Prothèse pour genou selon la revendication 1 ou 2,
**caractérisée en ce que**
le moyen de connexion ajustable de façon angulaire comprend un membre mâle comportant une base convexe sensiblement sphérique (37) et une partie saillante (36), la base convexe sensiblement sphérique étant adaptée pour être fixée à la prothèse du genou, ledit moyen de connexion ajustable de façon angulaire comprenant en outre un membre femelle sous forme d'un fourreau annulaire (38) adaptée pour retenir la partie saillante, le fourreau annulaire étant par ailleurs adaptée pour être reliée à un manchon de prothèse et/ou à une prothèse pour jambe et à un moyen de maintien ajustable (39) des deux membres dans une position angulaire choisie.

4. Prothèse pour genou selon la revendication 3,
**caractérisée en ce que**
ledit moyen de maintien ajustable (39) comprend un certain nombre de vis de serrage traversant la surface du fourreau annulaire (38) et destinées à s'engager dans la surface de la partie saillante (36) retenue dans cette cavité.

5. Prothèse pour genou selon la revendication 3 ou 4,
**caractérisée en ce que**
ladite partie saillante (36) a une forme frusto-pyramidale.

6. Prothèse pour genou selon l'une quelconque des revendications précédentes,
**caractérisée en ce que**
le dispositif de freinage (17) comprend un segment de frein (18) sous forme d'un disque ouvert et formant ainsi une paire de tiges (18a,18b) et coopérant avec un moyen d'activation du freinage sous forme d'un bras de levier (22) situé entre lesdites tiges (18a,18b) et qui après activation du dispositif de freinage force lesdites tiges à s'écarter, le moyen d'ajustement à l'usure (28,29) étant fixé au dit bras de levier activant le freinage (22).

7. Prothèse pour genou selon la revendication 6,
**caractérisée en ce que**
ledit moyen d'ajustement à l'usure comprend une cale de blocage (28) à ressort (29).

8. Prothèse pour genou selon la revendication 6 ou 7,
**caractérisée en ce que**
au moins un ressort (41) est agencé de façon à réagir d'après ledit moyen d'activation du freinage (21,22) pour écarter le bras de levier (22) de la position d'activation du freinage.
